(19) Europäisches Patentamt
European Patent Office
Office européen des brevets

(11) EP 0 587 087 B1

# (12) EUROPÄISCHE PATENTSCHRIFT

(45) Veröffentlichungstag und Bekanntmachung des Hinweises auf die Patenterteilung:
**16.06.1999 Patentblatt 1999/24**

(51) Int. Cl.$^6$: **A61K 31/215**, A61K 31/235, A61K 31/16, A61K 31/44, A61K 31/38

(21) Anmeldenummer: **93114260.8**

(22) Anmeldetag: **06.09.1993**

## (54) Substituierte Cyclohexan-Derivate zur Behandlung von Krankheiten

Substituted cyclohexane derivatives for the treatment of diseases

Dérivés du cyclohexane substitués pour le traitement de maladies

(84) Benannte Vertragsstaaten:
**AT BE CH DE DK ES FR GB GR IE IT LI LU MC NL PT SE**

(30) Priorität: **09.09.1992 DE 4230156**

(43) Veröffentlichungstag der Anmeldung:
**16.03.1994 Patentblatt 1994/11**

(73) Patentinhaber:
**HOECHST AKTIENGESELLSCHAFT
65929 Frankfurt am Main (DE)**

(72) Erfinder:
- **Hemmerle, Horst, Dr.
D-64653 Lorsch (DE)**
- **Schindler, Peter, Dr.
D-65812 Bad Soden (Taunus) (DE)**
- **Utz, Roland, Dr.
D-64409 Messel (DE)**
- **Rippel, Robert, Dr.
D-65719 Hofheim/Taunus (DE)**
- **Herling, Andreas, Dr.
D-65520 Bad Camberg (DE)**

(56) Entgegenhaltungen:
**EP-A- 0 163 270 WO-A-87/04619**

- **P.H. LIST et al. "Hagers Handbuch der Pharmazeutischen Praxis", 4. Ausgabe, Band 4, 1973, SPRINGER-VERLAG, Berlin Heidelberg, New York Seiten 415-418,186-228**
- **CHEMICAL ABSTRACTS, Band 104, Nr. 14, 7. April 1986, Columbus, Ohio, USA TAGUCHI, HEIHACHIRO et al. "Pharmaceuticals for treatment of influenza virus infection." Seite 402, Spalte 2, Zusammenfassung-Nr. 116 118q & Jpn. Kokai Tokkyo Koho JP 60-243 016 (85,243,016) (TSUMURA JUNTENDO, INC.)**
- **CHEMICAL ABSTRACTS, Band 95, 1981, Columbus, Ohio, USA ISTUDOR, VIORICA et al. "Study on the preparation of an "instant" type product (Ulcovex) for gastric and duodenal ulcers. Part I. Chemical study of Calendulae flores products, preparation of the "instant" type extract and determination of the control methodology." Seite 332, Spalte 2 - Seite 333, Spalte 1, Zusammenfassung-Nr. 86 207u**
- **CHEMICAL ABSTRACTS, Band 104, Nr. 17, 28. April 1986, Columbus, Ohio, USA JUCHI, SHIGERU et al. "Anti- allergic foods containing caffeic and/or caffetannie acid." Seite 543, Spalte 2, Zusammenfassung-Nr. 147 493n & Jpn. Kokai Tokkyo Koho JP 60-192 555 (85,192,555), (Osaka Yakuhin Kenkyusho K.K.)**
- **PLANTA MEDICA, Band 27, 1975, Hippokrates Verlag Stuttgart, V.I. LITVINENKO et al. ""Gerbstoffe" und Oxyzimtsäureabkömmlinge in Labiaten." Seiten 372-380**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 587 087 B1

- CHEMICAL & PHARMACEUTICAL BULLETIN, Band 34, Nr. 3, 1986 MAKOTO NISHIZAWA et al. "Isolation and Structural Elucidation of a New Lipoxygenase Inhibitor from Gardeniae Fructus" Seiten 1419-1421
- BIOCHIMICA ET BIOPHYSICA ACTA, Band 538, 1978, S.J.H. ASHCROFT et al. "Effects of Phloretin and Dextran-Linked Phloretin on Pancreatic Islet Metabolism and Insulin

## Beschreibung

Das Krankheitsbild des Diabetes ist durch erhöhte Blutzuckerwerte gekennzeichnet. Beim insulinpflichtigen oder Typ I Diabetes ist die Ursache das Absterben der insulinproduzierenden β-Zellen des Pankreas; die Behandlung erfolgt daher durch Insulingabe (Substitutionstherapie). Der nicht-insulinabhängige oder Typ II Diabetes ist dagegen durch eine verminderte Insulinwirkung auf Muskel- und Fettgewebe (Insulinresistenz) und eine gesteigerte Glucoseproduktion der Leber gekennzeichnet. Die Ursachen dieser Stoffwechselstörungen sind noch weitgehend ungeklärt. Die etablierte Therapie mit Sulfonylharnstoffen versucht die Insulinresistenz durch Steigerung der körpereigenen Insulinfreisetzung zu kompensieren, führt jedoch nicht in allen Fällen zu einer Normalisierung des Blutzuckerspiegels und vermag das Fortschreiten der Krankheit nicht aufzuhalten; viele Typ II Diabetiker werden schließlich durch "Erschöpfung" der β-Zellen insulinpflichtig und leiden an Spätschäden wie Katarakten, Nephropathien und Angiopathien.

Neue Therapieprinzipien zur Behandlung des Typ II Diabetes sind deshalb wünschenswert.

Die Konzentration der Blutglucose im nüchternen Zustand wird durch die Glucoseproduktion der Leber bestimmt. Verschiedene Arbeitsgruppen konnten zeigen, daß die Erhöhung der Blutzuckerwerte bei Typ II Diabetes mit einer proportional erhöhten Glucoseabgabe aus der Leber korrelieren. Die von der Leber in das Blut abgegebene Glucose kann sowohl durch Abbau von Leber-Glycogen (Glycogenolyse)

als auch durch Gluconeogenese gebildet werden.

Glucose-6-Phosphat ist das gemeinsame Endprodukt sowohl der Gluconeogenese als auch der Glykogenolyse. Der terminale Schritt der hepatischen Freisetzung von Glucose aus Glucose-6-Phosphat wird von der Glucose-6-Phosphatase (EC 3.1.3.9) katalysiert. Die Glucose-6-Phosphatase stellt einen im endoplasmatischen Reticulum (ER) vorkommenden Multienzym-Komplex dar. Dieser Enzym-Komplex besteht aus einer in der ER-Membran vorliegenden Glucose-6-Phosphat-Translocase, einer auf der luminalen Seite des endoplasmatischen Reticulum lokalisierten Glucose-6-Phosphatase und einer Phosphat-Translocase [für eine Übersicht siehe: Ashmore, J. und Weber G., "The Role of Hepatic Glucose-6-phosphatase in the Regulation of Carbohydrate Metabolism", in Vitamins and Hormones, Vol XVII (Harris R.S., Marrian G.F., Thimann K.V., Eds.), 92-132, (1959); Burchell A., Waddell I.D., "The molecular basis of the hepatic microsomal glucose-6-phosphatase system", Biochim. Biophys. Acta 1092, 129-137, (1990)]. Die vorliegende umfangreiche Literatur zeigt, daß unter allen untersuchten Bedingungen, die im Tierversuch zu erhöhten Blutglucose-Werten führen, z.B. Streptozotocin, Alloxan, Cortison, Thyroid-Hormone und Hungern, die Aktivität dieses Multienzym-Komplexes ebenfalls erhöht ist. Darüber hinaus weisen zahlreiche Untersuchungen darauf hin, daß die bei Typ II Diabetikern beobachtete erhöhte Glucose-Produktion mit einer erhöhten Glucose-6-Phosphatase-Aktivität verbunden ist. Die Bedeutung des Glucose-6-Phosphatase-Systems für eine normale Glucose-Homeostase wird ferner unterstrichen durch die hypoglykämischen Symptome von Patienten mit Glykogen-Speicherkrankheit Typ Ib, denen die Translocase-Komponente des Glucose-6-Phosphat-Systems fehlt.

Eine Verringerung der Glucose-6-Phosphatase-Aktivität durch geeignete Wirkstoffe (Inhibitoren) sollte zu einer entsprechend verringerten hepatischen Glucose-Freisetzung führen. Diese Wirkstoffe sollten in der Lage sein, die Glucose-Produktion der Leber dem effektiven peripheren Verbrauch anzupassen. Die dadurch im nüchternen Zustand von Typ II Diabetikern gesenkten Blutglucose-Werte dürften darüber hinaus auch eine präventive Wirkung im Hinblick auf diabetische Spätschäden haben.

In der Literatur ist eine Reihe von unspezifischen Inhibitoren der Glucose-6-Phosphatase beschrieben worden wie z.B. Phlorhizin [Soodsma, J. F., Legler, B. und Nordlie, R. C., J. Biol. Chem. 242, 1955-1960, (1967)], 5,5'-Dithio-bis-2-nitrobenzoesäure [Wallin, B. K. und Arion, W. J., Biochem. Biophys. Res. Commun. 48, 694-699, (1972)], 2,2'-Di-isothiocyanato-stilben und 2-Isothiocyanato-2'-acetoxy-stilben [Zoccoli, M. A. und Karnowski, M. L., J. Biol. Chem. 255, 1113-1119, (1980)]. Bisher liegen jedoch noch keine therapeutisch verwertbaren Inhibitoren des Glucose-6-Phosphatase-Systems vor.

Substituierte Cyclohexan-Derivate, die nachfolgend näher definiert sind, sind aus der chemischen und biologischen Literatur teilweise bekannte Verbindungen, die aus zahlreichen Pflanzen isoliert werden konnten (R. Krasemann, Arch. Pharm. 293, 721 (1960)). Über pharmakologische und biochemische Wirkungen dieser Ester ist aber nur wenig bekannt. Chlorogensäure, ein typischer Vertreter der hier angesprochenen Verbindungen, ist unter anderem als Hemmer der Lipoxygenase (M. Nishizawa et al., Chem. Pharm. Bull, 34(3), 1419 (1986)) beschrieben worden.

Wir haben nun gefunden, daß bestimmte Ester von substituierten Cyclohexancarbonsäuren, wie z. B. die Chlorogensäure (Nr. 17 der von uns untersuchten Verbindungen) Hemmer des Glucose-6-Phosphatase-Systems sind.

Die Erfindung betrifft die Anwendung von Cyclohexan-Derivaten der Formel I

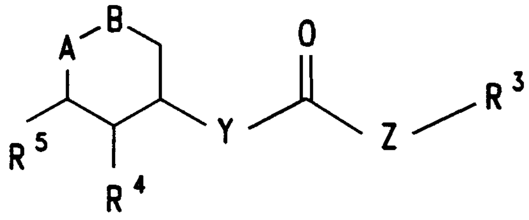

I

worin

A-B die Gruppe 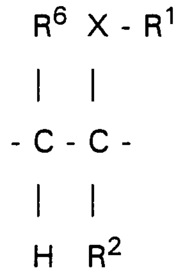

oder

die Gruppe 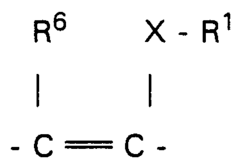

bedeutet,

- $R^1$: CN, COOH, COO-($C_1$-$C_4$-Alkyl), $C_1$-$C_4$-Alkanoyl, $SO_3$-($C_1$-$C_4$-Alkyl), $SO_3H$, $PO(OH)_2$, PO(OH)(O-$C_1$-$C_4$-Alkyl) oder $PO(O$-$C_1$-$C_4$-$Alkyl)_2$ ist,
- $R^2$: H, OH oder F ist,
- $R^3$: H, Phenyl, Naphthyl, Pyridyl, Thienyl, Furyl ist, wobei der Aromat bzw. Heteroaromat ein- oder mehrfach mit F, Cl, Br, I, OH, $NO_2$, $C_1$-$C_4$-Alkanoyl, $C_1$-$C_4$-Alkoxy, $C_1$-$C_4$-Alkyl, Phenyl, Phenoxy, Thienyl, Furyl, Pyridyl, Imidazolyl oder Benzyloxy substituiert sein kann, wobei die Substituenten gleich oder verschieden sind,
- $R^4$, $R^5$, $R^6$: H, OH, F, Cl, Br, $C_1$-$C_4$-Alkanoyl, $C_1$-$C_4$-Alkyl, Phenyl, Phenoxy, Thienyl, Furyl, Pyridyl, Imidazolyl oder Benzyloxy bedeuten, wobei $R^4$, $R^5$, $R^6$ gleich oder verschieden sind,
- X: $-(CH_2)_n-$, -CH = CH- oder $-CH_2OCH_2-$ ist,
- Y: $-(CH_2)_n-$, O, S oder NH ist,
- Z: $-(CH_2)_n-$ oder -CH = CH- ist und
- n: null, 1, 2, 3 oder 4 ist

zur Herstellung von Arzneimitteln zur Behandlung des Typ II Diabetes und anderer Erkrankungen, die durch einen erhöhten Glucose-Ausstoß aus der Leber oder eine erhöhte Aktivität des Glucose-6-Phosphatase-Systems gekennzeichnet sind.

Bevorzugt ist die Anwendung solcher Verbindungen der Formel I, in denen die Reste die folgende Bedeutung haben:

- $R^1$: COOH, COO-($C_1$-$C_4$-Alkyl), $PO(OH)_2$, PO(OH)(O-$C_1$-$C_4$-Alkyl) oder $PO(O$-$C_1$-$C_4$-$Alkyl)_2$,
- $R^2$: H oder OH
- $R^3$: H, Phenyl, Naphthyl, Pyridyl, Thienyl, Furyl, wobei der Aromat bzw. Heteroaromat ein-, zwei- oder dreifach mit F, Cl, Br, I, $NO_2$, OH, $C_1$-$C_4$-Alkanoyl, $C_1$-$C_4$-Alkoxy, $C_1$-$C_4$-Alkyl, Phenyl, Phenoxy, Thie-

nyl, Furyl, Pyridyl, Imidazolyl oder Benzyloxy substituiert sein kann, wobei die Substituenten gleich oder verschieden sind,

$R^4$, $R^5$ u. $R^6$ : H, OH, F, Cl, Br, $C_1$-$C_4$-Alkanoyl, $C_1$-$C_4$-Alkyl, Phenyl, Phenoxy, Thienyl, Furyl, Pyridyl, Imidazolyl oder Benzyloxy, wobei $R^4$, $R^5$, $R^6$ gleich oder verschieden sind,

X: $-(CH_2)_n-$, -CH = CH- oder $-CH_2OCH_2-$,

Y: $-(CH_2)_n-$, O, S oder NH,

Z: $-(CH_2)_n-$ oder -CH = CH- und

n: null, 1, 2, 3 oder 4

Besonders bevorzugt ist die Anwendung solcher Verbindungen der Formel I, in denen Reste die folgende Bedeutung haben:

$R^1$: COOH oder COO-($C_1$-$C_4$-Alkyl)

$R^2$: H oder OH,

$R^3$: H, Phenyl, Naphthyl, Pyridyl, Thienyl, Furyl, wobei der Aromat bzw. Heteroaromat ein-, zwei- oder dreifach mit F, Cl, OH, $NO_2$, $C_1$-$C_4$-Alkanoyl, $C_1$-$C_4$-Alkoxy, $C_1$-$C_4$-Alkyl, Phenyl, Phenyloxy oder Benzyloxy gleich oder verschieden substituiert sein kann,

$R^4$, $R^5$ und $R^6$ : H oder OH, wobei $R^4$, $R^5$, $R^6$ gleich oder verschieden sind,

X: $-(CH_2)_n-$ und n = null, 1 oder 2

Y: O oder NH,

Z: $-(CH_2)_n-$ mit n = 0 oder 2 oder -CH = CH-,

Die in den Verbindungen der Formel I vorhandenen Alkyl- Alkoxy- und Alkanoylreste sind geradkettig oder verzweigt.

Die Wirkung der erfindungsgemäßen Verbindungen auf das Glucose-6-Phosphatase-Sytem wurde in einem Enzymtest in Lebermikrosomen untersucht.

Für die Präparation der die Glucose-6-Phosphatase enthaltenden Mikrosomen-Fraktion wurden frische Leber-Organe von männlichen Wistar-Ratten verwendet und wie in der Literatur beschrieben aufgearbeitet [Canfield, W. K. und Arion, W. J., J. Biol. Chem. 263, 7458-7460, (1988)]. Diese Mikrosomen-Fraktion kann bei -70 °C mindestens 2 Monate lang ohne signifikanten Aktivitätsverlust aufbewahrt werden.

Der Machweis der Glucose-6-Phosphatase-Aktivität wurde wie in der Literatur angegeben (Arion, W. J. in Methods Enzymol. 174, Academic Press 1989, Seite 58-67) durch Bestimmung des aus Glucose-6-Phosphat freigesetzten Phosphats durchgeführt. 0,1 ml Testansatz enthielten Glucose-6-Phosphat (1 mMol/l), die Testsubstanz, 0,1 mg Mikrosomen-Fraktion und 100 mMol/l HEPES-Puffer (4-(2-Hydroxyethyl)piperazin-1-ethansulfonsäure), pH 7,0. Die Reaktion wurde durch Zugabe des Enzyms gestartet. Nach Ablauf von 20 min bei Raumtemperatur wurde die Reaktion durch Zugabe von 0,2 ml Phosphat-Reagens gestoppt. Die Probe wurde 30 min lang bei 37 °C inkubiert, und die Absorption (A) der blauen Farbe anschließend bei 570 nm gemessen. Die inhibitorische Wirksamkeit der Testsubstanz ergab sich durch Vergleich mit einer Kontroll-Reaktion, die keine Testsubstanz enthielt nach der Formel

$$\text{Prozent Hemmung} = \frac{\text{A(Kontrolle) - A(Testsubstanz)}}{\text{A(Kontrolle)}}\ 100$$

In Tabelle 1-3 sind beispielhaft die für eine Reihe der Verbindungen der Formel I erhaltenen Hemmwerte aufgeführt. Die Herstellung ist in den Ausführungsbeispielen beschrieben.

Tabelle 1

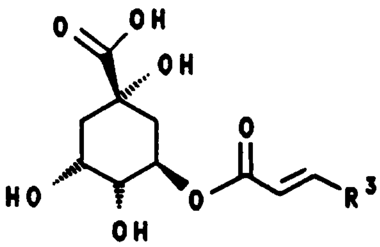

| $R^3$ | Konzentration [M] | Hemmung [%] | Verbindung Nr. |
|---|---|---|---|
| 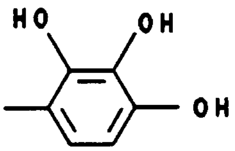 | $3.1 \cdot 10^{-4}$ | 50 | 1 |
| 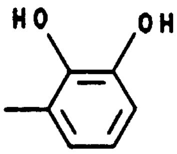 | $3.1 \cdot 10^{-4}$ | 50 | 2 |
| 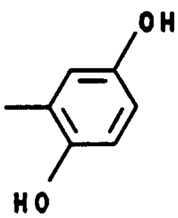 | $6.8 \cdot 10^{-4}$ | 50 | 3 |

| | | | |
|---|---|---|---|
| 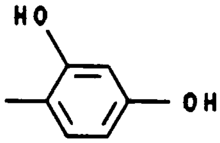 | $6.8 \cdot 10^{-4}$ | 50 | 4 |
| 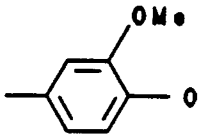 | $4.1 \cdot 10^{-4}$ | 50 | 5 |
| 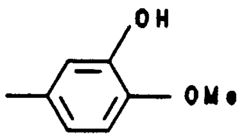 | $4.6 \cdot 10^{-4}$ | 50 | 6 |
| 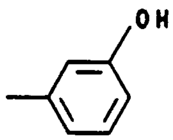 | $5.3 \cdot 10^{-4}$ | 50 | 7 |
| 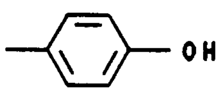 | $2.6 \cdot 10^{-4}$ | 50 | 8 |
| 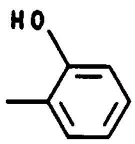 | $4.8 \cdot 10^{-4}$ | 50 | 9 |

| | | | |
|---|---|---|---|
| 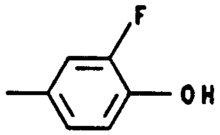 | $1 \cdot 10^{-3}$ | 50 | 10 |
| 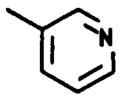 | $1 \cdot 10^{-3}$ | 20 | 11 |
| 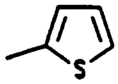 | $3.8 \cdot 10^{-4}$ | 50 | 12 |
| 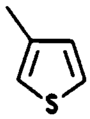 | $7.8 \cdot 10^{-4}$ | 50 | 13 |
| 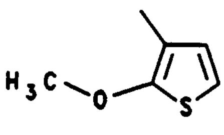 | $1 \cdot 10^{-3}$ | 20 | 14 |
| 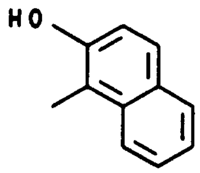 | $4.6 \cdot 10^{-4}$ | 50 | 15 |
| 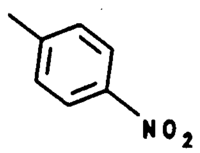 | $1 \cdot 10^{-3}$ | 35 | 16 |

| | | | |
|---|---|---|---|
| 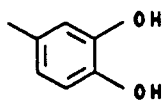 | $2.3 \cdot 10^{-4}$ | 50 | 17 |
| 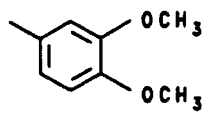 | $6.8 \cdot 10^{-4}$ | 50 | 17*a* |

Tabelle 2

| Struktur | Konzentration [M] | Hemmung [%] | Verbindung Nr. |
|---|---|---|---|
| 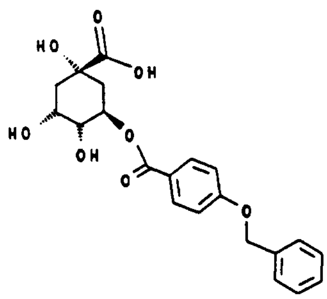 | $6.8 \cdot 10^{-4}$ | 50 | 18 |
| 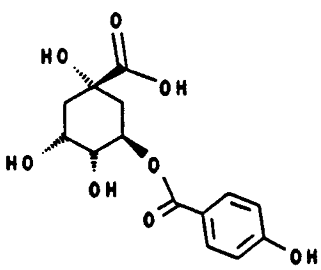 | $9.2 \cdot 10^{-4}$ | 50 | 19 |

Tabelle 3

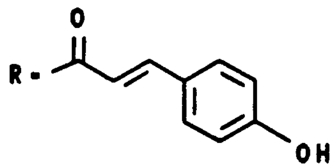

| Struktur | Konzentration [M] | Hemmung [%] | Verbindung Nr. |
|---|---|---|---|
| 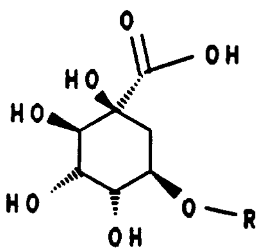 | $1 \cdot 10^{-3}$ | 10 | 20*a* |
| 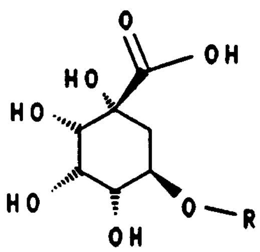 | $1 \cdot 10^{-3}$ | 30 | 20 |
| 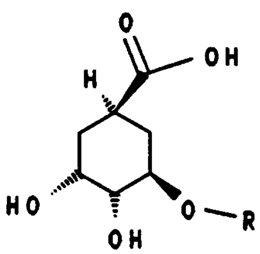 | $2.3 \cdot 10^{-4}$ | 50 | 21 |

| | | | |
|---|---|---|---|
| 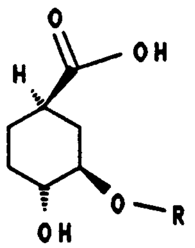 | 1·10$^{-3}$ | 20 | 22 |
| 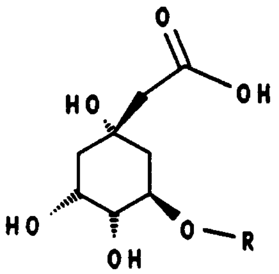 | 1·10$^{-3}$ | 30 | 23 |
| 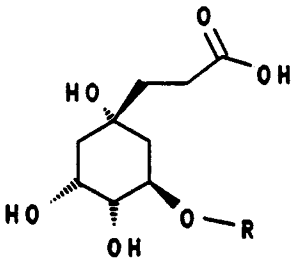 | 1·10$^{-3}$ | 45 | 24 |
| 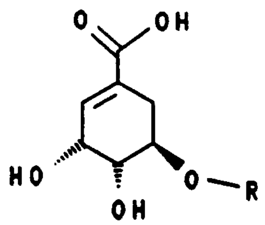 | 2.5·10$^{-4}$ | 50 | 25 |

| | | | |
|---|---|---|---|
| 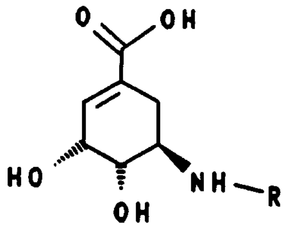 | $1 \cdot 10^{-3}$ | 15 | 26 |
| 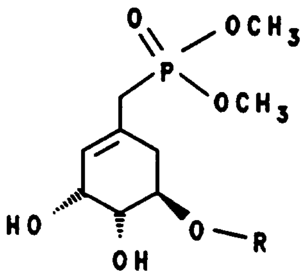 | $1 \cdot 10^{-3}$ | 15 | 27 |
| 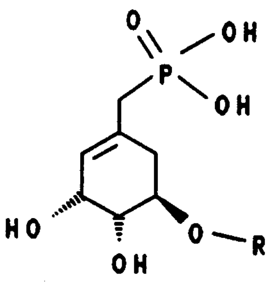 | $1 \cdot 10^{-3}$ | 28 | 28 |

In den Arzneimitteln gemäß vorliegender Erfindung, die nach üblichen Verfahren hergestellt werden, können neben Verbindungen der Formel I auch pharmazeutisch unbedenkliche Zusatzstoffe, wie Verdünnungsmittel und/oder Trägermaterialien, enthalten sein. Hierunter sind physiologisch unbedenkliche Substanzen zu verstehen, die nach dem Vermischen mit dem Wirkstoff diesen in eine für die Verabreichung geeignete Form bringen.

Die orale Verabreichung ist bevorzugt.

Geeignete feste oder flüssige galenische Zubereitungsformen sind beispielsweise Tabletten. Dragees, Pulver, Kapseln, Sirupe, Emulsionen, Suspensionen, Tropfen sowie Präparate mit protrahierter Wirkstoff-Freigabe. Als häufig verwendete Trägerstoff bzw. Verdünnungsmittel seien z. B. verschiedene Zucker oder Stärkearten, Cellulosederivate, Magnesiumcarbonat, Gelatine, tierische und pflanzliche Öle, Polyethylenglykole, Wasser oder andere geeignete Lösungsmittel sowie wesserhaltige Puffermittel, die durch Zusatz von Salzen isotonisch gemacht werden können, genannt. Außerdem können gegebenenfalls oberflächenaktive Mittel, Farb- und Geschmacksstoffe, Stabilisatoren, sowie Konservierungsmittel als weitere Zusatzstoffe in den erfindungsgemäßen Arzneimittelzubereitungen Verwendung finden.

Vorzugsweise können Präparate in Dosierungseinheiten hergestellt werden. Insbesondere stellen Tabletten und Kapseln Beispiele für geeignete Dosierungseinheiten dar. Jede Dosierungseinheit, insbesondere für die orale Applikation, kann bis zu 500 mg, bevorzugt jedoch 10 bis 200mg, des aktiven Bestandteils enthalten. Jedoch können auch darüber oder darunter liegende Dosierungseinheiten verwendet werden, die gegebenenfalls vor Verabreichung zu teilen bzw. zu vervielfachen sind. Gegebenenfalls können die Dosierungseinheiten für die orale Verabreichung mikroverkapselt werden, um die Abgabe z.B. zu verzögern. Eine kontrollierte Freigabe erreicht man beispielsweise auch durch Überziehen oder Einbetten von teilchenförmigem Material in geeignete Polymere, Wachse oder dergleichen.

Die untersuchten Verbindungen wurden wie nachfolgend beschrieben synthetisiert.

## Formelschema 1

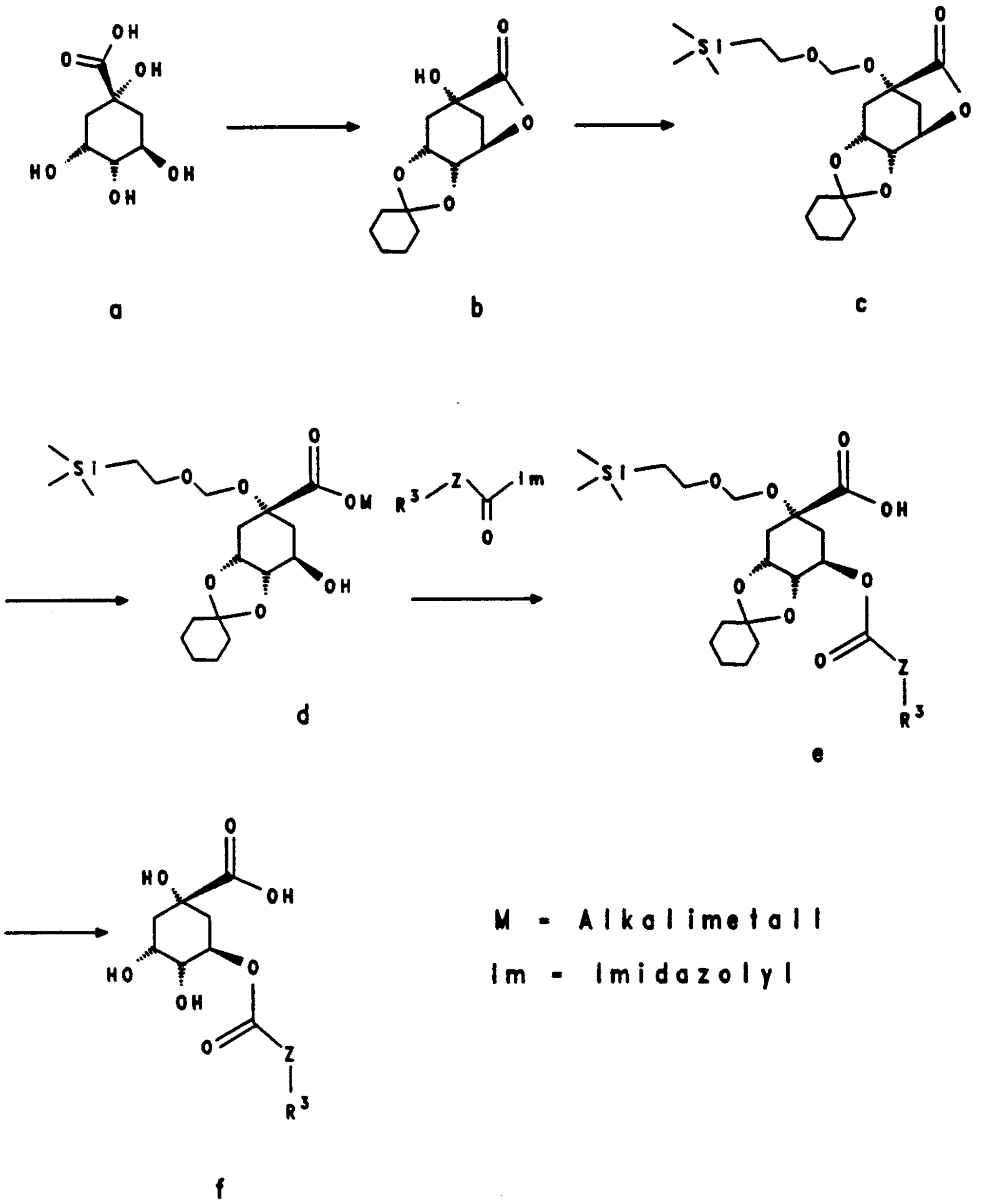

a b c

d e

f

M = Alkalimetall

Im = Imidazolyl

Darstellung von b aus a:

163.3 g (0.85 mol) Verbindung a (Fischer, Dangschat, Chem. Ber. 65, 1009 (1932)) wurden in 186 ml (1.8 mol) Cyclohexanon suspendiert und es wurden 0.5 ml konz. Schwefelsäure zugesetzt. Anschließend erhitzte man langsam auf 200°C Heizbad-Temperatur und destillierte ein Wasser/Cyclohexanon-Azeotrop ab. Nachdem kein Azeotrop mehr überdestillierte, wurde die hellbraune Reaktionslösung weitere 2 h bei 200°C Badtemperatur gerührt. Danach ließ man die Reaktionslösung auf 70°C abkühlen und gab 10 g Natriumhydrogencarbonat zu. Anschließend versetzte man mit 700 ml Essigsäureethylester, wusch die organische Phase mit Wasser und gesättigter Natriumchlorid-Lösung. Danach engte man die organische Phase im Vakuum ein. Den hellgelben Rückstand kristallisierte man aus Isopropanol/Wasser 1:1 und erhielt 142.1 g (75%) Lacton b als farblose Kristalle. F.p.: 140-141°C.

Darstellung von c aus b:

38.14 g (0.15 mol) Hydroxylacton b wurden in 180 ml Dichlormethan gelöst. Es wurden 53.0 ml (0.3 mol) Diisopropylethylamin zugesetzt. Zu dieser Lösung tropfte man bei Raumtemperatur 45.0 ml (0.254 mol) Trimethylsilylethyloxymethylchlorid und rührte 6 h bei Rückfluß-Temperatur. Anschließend wurde die Reaktionslösung auf gesättigte Ammoniumchlorid-Lösung gegeben und mit Essigester extrahiert. Die vereinigten organischen Phasen extrahierte man mit ca. 6 °C kalter 1 N Kaliumhydrogensulfat-Lösung und trocknete mit Natriumsulfat. Nach dem Einengen im Vakuum erhielt man einen hellgelben Rückstand, der aus Heptan/EE 6:1 kristallisiert wurde. Man erhielt 57.0 g (98%) c.
F.p.: 100-102 °C

Darstellung von d aus c:

1.38 g (3.6 mol) c wurden in 8 ml Dioxan gelöst. Nach Zugabe von 0.4 ml Wasser wurden bei Raumtemperatur 3.8 ml 1 N Natronlauge zugetropft. Man rührte die Reaktionsmischung 2 h und engte anschließend im Vakuum ein. Man erhielt 1.3 g (85%) d als amorphen Feststoff.
$^1$H-NMR (270 MHz, $d_6$-DMSO): d = 0.01 ppm (s, 9H), 0.72-0.89 (m, 2 H), 1.21-1.62 (m, 10 H), 1.65-1.78 (m, 1H), 1.82-1.92 (m, 1H), 1.94-2.08 (m, 2 H), 3.38-3.63 (m, 3H), 3.82-3.88 (m, 1H), 4.18-4.27 (m, 1H), 4.61-4.72 (m, 2H), 7.80-7.90 (m, 1H).
Die Stufen d, e und f werden exemplarisch mit der Darstellung von Verbindung 8 beschrieben.

Darstellung von Verbindung 8:

## Formelschema 2

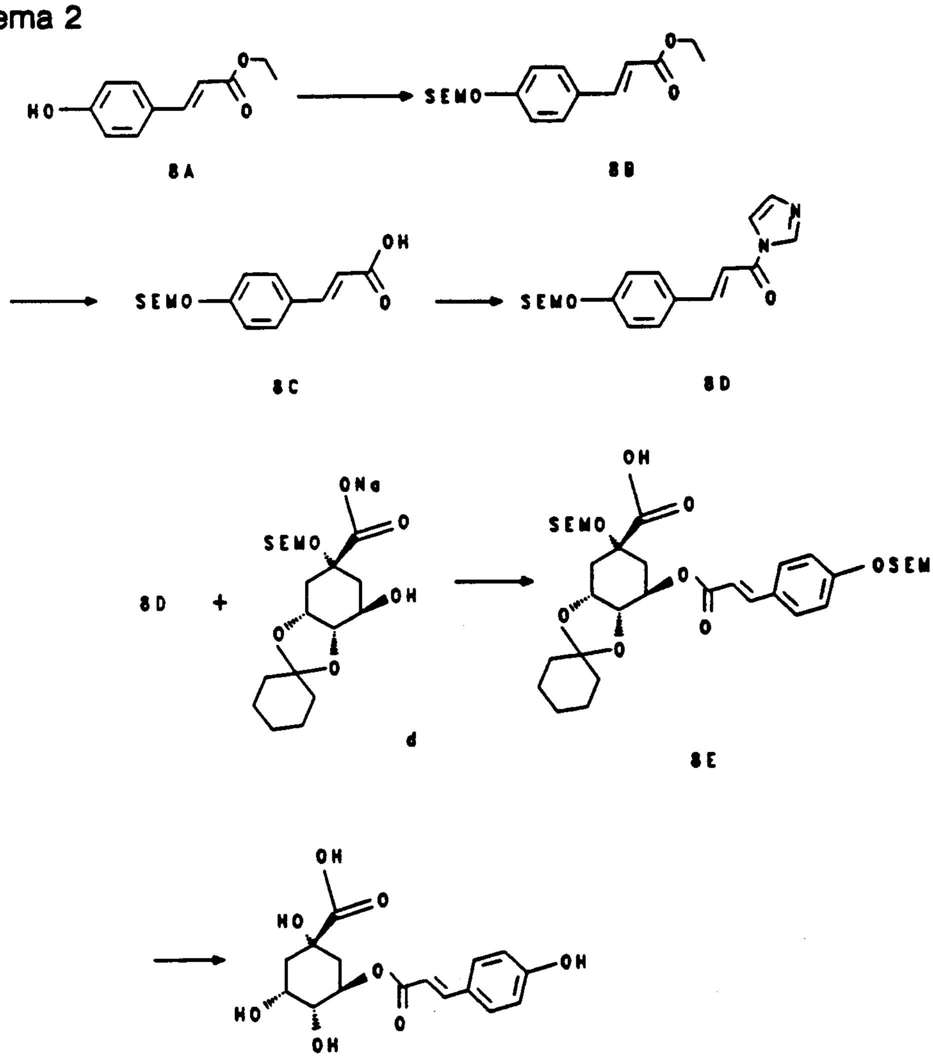

8A 8B 8C 8D d 8E

8

SEM = Trimethylsilyloxymethyl-

Darstellung von 8C (aus 8A über 8B)

10.0 g (0.052 mol) p-Hydroxyzimtsäureester (8A) wurden in 60 ml wfr. Dichlormethan gelöst. Es wurden 27 ml (0.156 mol) Diisopropylethylamin zugesetzt und bei Raumtemperatur unter Argon-Atmosphäre 19.5 ml (0.11 mol) Trimethylsilylethoxymethylchlorid zugetropft. Man rührte 4 h bei Raumtemperatur und goß anschließend die Reaktionslösung auf eisgekühlte Ammoniumchlorid-Lösung. Man extrahierte mit Essigsäureethylester, wusch die vereinigten organischen Phasen nacheinander mit eiskalter 1 N Kaliumhydrogensulfat-Lösung und gesättigter Natriumchlorid-Lösung. Nach dem Trocknen der organischen Phase mit Natriumsulfat engte man im Vakuum ein. Man erhielt 16.8 g Ether 8B, der ohne weitere Reinigung in 600 ml Dioxan gelöst und bei Raumtemperatur mit 160 ml (0.8 mol) 5 N Natronlauge versetzt wurde. Nach 24 h wurde das Methanol im Vakuum abdestilliert, und die wässrige Suspension des Natrium-Salzes von 8C mit 2 N Salzsäure auf pH 4 angesäuert. Die Säure 8C fiel nahezu quantitativ aus und konnte abgesaugt und mit Wasser gewaschen werden. Man erhielt 16.02 g 8C. F.p.: 93-96°C.

Darstellung von 8E aus 8C und d:
( entspricht im allgemeinen Schema 1 der Stufe e)

a) 7.95 g (27 mmol) 8C wurden in 35 ml wfr. Dimethylformamid gelöst. Bei Raumtemperatur wurde eine Lösung von 4.54 g (27 mmol) Carbonyldiimidazol gelöst in 35 ml wfr. Dimethylformamid zugetropft. Anschließend erwärmte man diese Lösung 1 h auf 60-70°C, wobei eine $CO_2$-Entwicklung zu beobachten war.
b) Zu einer Lösung von 8.92 g (0.021 mol) Natriumsalz d in 50 ml wasserfreiem Dimethylformamid wurden bei Raumtemperatur unter Argon-atmosphäre 0.75 g (0.025 mol) Natriumhydrid (80%ig) gegeben. Man rührte diese Suspension 1 h bei Raumtemperatur und gab danach bei 0-5°C die gemäß a) hergestellte Lösung des Imidazolids 8D zu. Man rührte die Lösung 2.5 h bei O-5°C und gab anschließend den Reaktionsansatz auf gesättigte Ammoniumchlorid-Lösung. Durch Zugabe von 1 N Kaliumhydrogensulfat-Lösung wurde die Mischung auf pH 4 angesäuert und mit Essigsäureethylester extrahiert. Die vereinigten organischen Phasen wurden nacheinander mit gesättigter Ammoniumchlorid-Lösung, Wasser und gesättigter Natriumchlorid-Lösung gewaschen. Die organische Phase trocknete man mit Natriumsulfat, engte sie im Vakuum ein und chromatographierte den öligen Rückstand an Kieselgel (Laufmittel: Essigsäurethylester/n-Heptan/Eisessig 20:60:1). Man erhielt 10.3 g (78%) 8E als farbloses Öl.

$^1$H-NMR (270 MHz, $CDCl_3$) : d= 0.02 ppm (s, 9H), 0.05 (s, 9H), 0.91-1.03 (m, 4H), 1.5-1.78 (m, 10H), 1.91-2.05 (m, 1H), 2.28-2.42 (m, 2H), 2.57-2.63 (m, 1H), 3.68-3.90 (m, 4H), 4.14-4.20 (m, 1H), 4.42-4.52 (m, 1H), 4.91-4.96 (m, 1H), 5.11-5.18 (m, 1H), 5.24 (s, 2H), 5.21-5.34 (m, 1H), 6.32 (d, J=10 Hz, 1H), 7.02-7.08 (m, 2H), 7.42-7.5 (m, 2H), 7.65 (d, J=10 Hz, 1H), 13 (s, br, COOH, 1H).

Darstellung von 8 aus 8E:
(entspricht im allegemeinen Schema 1 der Stufe f)

5.02 g (7.4 mmol) 8E wurden in 130 ml Dioxan gelöst und bei Raumtemperatur unter Rühren mit 95 ml (0.19 mol) 2 N Salzsäure versetzt. Man rührte 20 h bei Raumtemperatur. Nach Ende der Reaktion wurde die klare Lösung mit 2 N Natronlauge auf pH 3-4 eingestellt und im Vakuum eingeengt. Der feste Rückstand wurde in Essigsäureethylester/Methanol 3:1 verrührt und das unlösliche Natriumchlorid abfiltriert. Das Filtrat engte man erneut ein und chromatographierte den Rückstand an Kieselgel (Esigsäurethylester/Methanol/Wasser/Eisessig 100/10/10/5). Man erhielt 1.95 g (70%) 8. F.p.: 235-238°C.

Die in Tabelle 4 angegeben Beispiele (Verbindungen) wurden nach dem obigen Verfahren dargestellt. Dabei unterschied sich die Synthese der Hydroxy-Gruppen enthaltenden Verbindungen im Rest $R^3$ der allgemeinen Formel I durch die entsprechende Schutzgruppen-Operationen von den übrigen, bei denen diese nicht notwendig waren.

In den nachfolgenden Tabellen 4 und 5 wurden die physikalischen Daten der synthetisierten Beispiele zusammengefaßt.

Tabelle 4

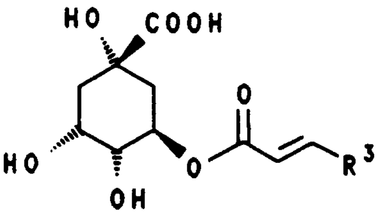

| $R^3$ | Physikalische Daten (NMR/MS bzw. Schmelzpunkt) | Verbindung Nr. |
|---|---|---|
| 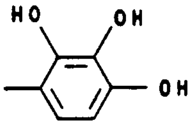 | F.p.: 105-110°C | 1 |

| $R^3$ | Physikalische Daten (NMR/MS bzw. Schmelzpunkt) | Verbindung Nr. |
|---|---|---|
| 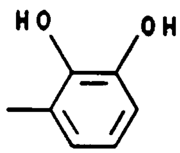 | $^1$H-NMR (270 MHz, $d_6$-DMSO): d=1.72-2.08 (m,4H),3.51-3.62(m,1H),3.91-3.99(m,1H),4.7-4.95 (m,2H),5.08-5.17(m,1H),6.49(d,J=10 Hz,1H), 6.61-6.70(m,1H),6.69-6.88(m,1H),6.98-7.08 (m,1H), 7.82(d,J=10 Hz,1H),9-10(s,br,2H),12-13(s,br,1H) | 2 |
| 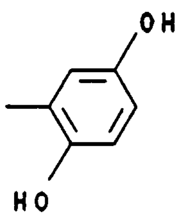 | MS(Cl)=355.7(M+H$^+$) | 3 |
| 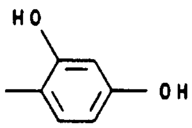 | F.p.:180°C | 4 |
| 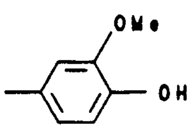 | F.p.:110-120°C | 5 |
| 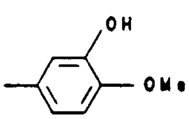 | F.p.:166-169°C | 6 |

| $R^3$ | Physikalische Daten (NMR/MS bzw. Schmelzpunkt) | Verbindung Nr. |
|---|---|---|
| 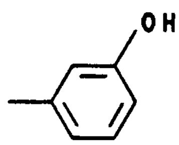 | $^1$H-NMR(270 MHz,$d_6$-DMSO):d=1.72-2.10(m,4H), 3.21-3.60(m,3H),3.90-4.00(m,1H),4.70-4.82 (m,1H),4.85-4.95(m,1H),5.05-5.15(m,1H),6.37 (d,J=10 Hz,1H), 6.81-6.85(m,1H),7.0-7.05 (m,1H), 7.09-7.15(m,1H),7.18-7.27(m,1H),7.5(d,J=10 Hz, 1H), 9.6(s,br,2H),11-13(COOH,1H) | 7 |
| 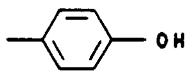 | F.p.:235-238°C (Zersetzung) | 8 |
| 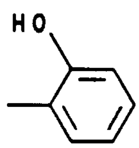 | F.p.:105-110°C | 9 |
| 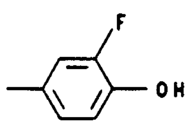 | F.p.:208-211°C<br>MS(Cl)=357(M+H$^+$) | 10 |
| 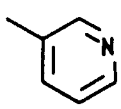 | F.p.:195-200°C(Zersetzung | 11 |
| 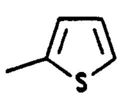 | F.p.:85-95°C | 12 |

| $R^3$ | Physikalische Daten (NMR/MS bzw. Schmelzpunkt) | Verbindung Nr. |
|---|---|---|
| 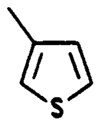 | $^1$H-NMR(270 MHz,$d_6$-DMSO):d=1.72-2.10(m,4H), 3.1-3.7(m,3H),3.90-4.00(m,1H),4.7-5.0 (m,2H),5.06-5.13(m,1H),6.32(d,J=10 Hz,1H), 7.52-7.65(m,3H),7.95-8.02(m,1H), MS(Cl)-329.1(M+$H^+$) | 13 |
| 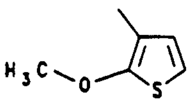 | F.p.:178-181°C | 14 |
| 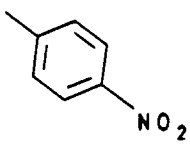 | F.p.:180-185°C | 16 |
| 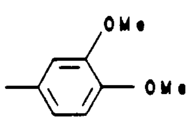 | $^1$H-NMR(270 MHz,$d_6$-DMSO):d=1.73-2.10(m,4H), 3.25-3.41(m,1H),3.52-3.62(m,1H),3.78(s,3H),3.82 (s,3H),3.91-4.99(m,1H),4.72-4.83(m,1H),4.86-4.92(m,1H),5.05-5.18(m,1H),6.45(d,J=10 Hz,1H), 6.97-7.03(m,1H),7.18-7.27(m,1H),7.30-7.36 (m,1H),7.52(d,J=10 Hz, 1H), 12.5(s,br,2H), | 17*a* |
| 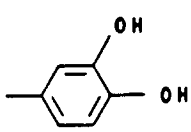 | F.p.:208-210°C | 17 |

Tabelle 5

| Struktur | Schmelzpunkt | Verbindung Nr. |
|---|---|---|
| 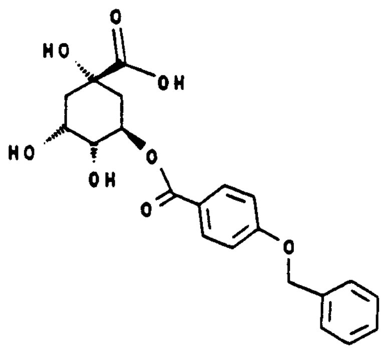 | 165-170°C | 18 |
| 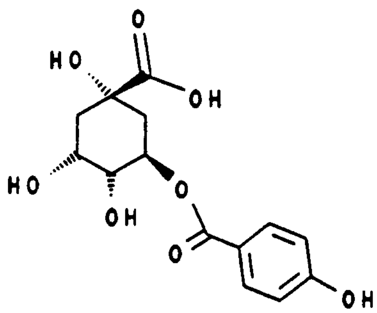 | 235-240°C (Zersetzung) | 19 |

Darstellung der Verbindung 20 bzw. 20α:

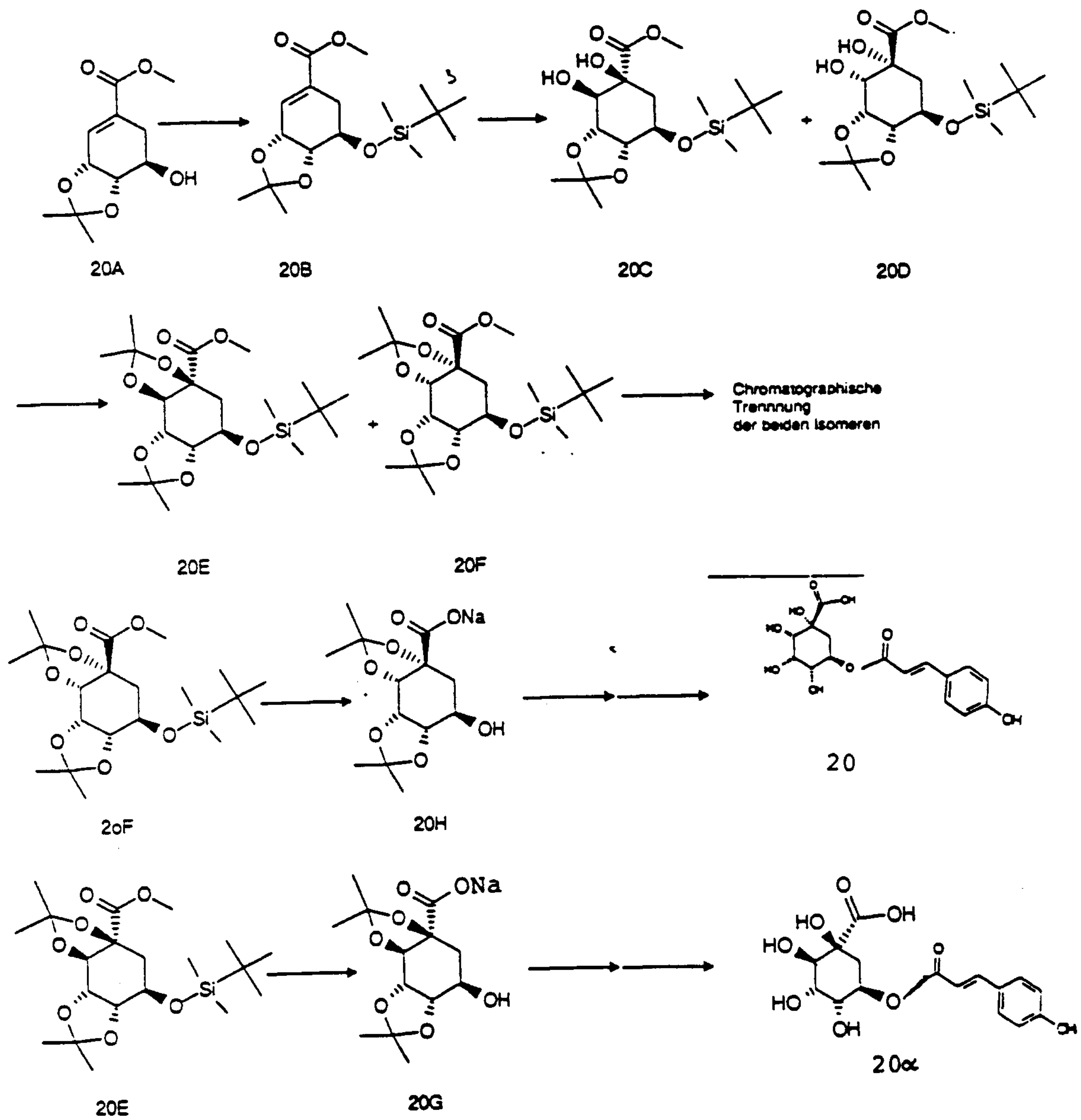


20A 20B 20C 20D

20E 20F

2oF 20H 20

20E 20G 20α

Darstellung von 20B aus 20A:

4.0 g (17.5 mmol) der bekannten Verbindung 20A (S.A. Bowles et al., Tetrahedron 46, 3981(1990)) wurden in 30 ml wasserfreiem Dimethylformamid gelöst. Es wurden 1.61 g (23.7 mmol) Imidazol sowie 2.64 g (12.5 mmol) t-Butyldimethylsilylchlorid zugegeben. Nach 12 h bei 25°C wurde die Reaktionslösung mit 200 ml gesättigter Ammoniumchlorid-Lösung versetzt und mit 300 ml Methyl-t-butylether portionsweise extrahiert. Die vereinigten organischen Phasen wurden mit Wasser und gesättigter Natriumchlorid-Lösung gewaschen und mit Magnesiumsulfat getrocknet. Man erhielt 5.4 g (90%) 20B als farbloses Öl.

$^1$H-NMR (270MHz, $CDCl_3$): δ=0.06 ppm (s, 3H), 0.09 (s, 3H), 0.76 (s, 9H), 1.39 (s, 6H), 2.23-2.40 (m, 1H), 2.48-2.62 (m, 1H), 3.76 (s, 3H), 4.0-4.12 (m, 2H), 4.66-4.72 (m, 1H), 6.80-6.86 (m, 1H).

Darstellung von 20C bzw. 20D aus 20B:

5.4 g (15.8 mmol) 20B wurden in 100 ml t-Butanol gelöst. Es wurden 1.9 g (25.3 mmol) Trimethylamin-N-oxid sowie 20 ml Wasser zugegeben. Anschließend fügte man 100 mg (0.4 mmol) Osmiumtetroxid komplexiert an 2.0 g Polyvinylpyridin zu und rührte 14 h bei Siedetempertur. Danach filtrierte man den Katalysator ab, engte das Filtrat ein und chromatographierte den Rückstand an Kieselgel (Laufmittel Essigester/n-Heptan 1:1). Man erhielt 2.5 g (42%) 20C/20D im Verhältnis 3:1 als farbloses Öl.

Mischung der beiden Isomeren 20C/20D:

$^1$H-NMR (270MHz, $CDCl_3$): δ=0.08-0.14 (m, 6H),0.88-0.92 (m, 9H), 1.38-1.40 (m, 3H), 1.51-1.55 (m, 3H), 1.80-2.0 (m, 1H), 2.28-2.48 (m, 1H), 3.61-4.52 (m, 7H).

Darstellung von 20E und 20 F aus 20C bzw. 20D:

2.5 g (6.6 mmol) einer 3:1-Mischung von 20C/20D wurden in 60 ml wasserfreiem Dichlormethan gelöst. Es wurden 5 ml 2,2-Dimethoxypropan sowie 200 mg Pyridinium-p-toluolsulfonat zugegeben. Man erhitzte die Reaktionslösung 6 h auf Siedetemperatur und engte anschließend die Lösung im Vakuum ein. Der Rückstand, eine Mischung aus 20E und 20F, wurde an Kieselgel aufgetrennt (Laufmittel Essigester/n-Heptan 3:1) und man erhielt insgesamt 2.4 g (87%) 20E und 20F jeweils als farblose Öle.

$^1$H-NMR (270 MHz, $CDCl_3$): δ= 0.08 ppm (s, 3H), 0.09 (s, 3H), 0.90 (s, 9H), 1.34 (s, 3H), 1.39 (s, 3H), 1.45 (s, 3H), 1.50 (s, 3H), 1.72 (dd, J=13.5, J=12 Hz, 1H), 2.19 (dd, J=4.0, J=14.5 Hz, 1H), 3.81 (s, 3H), 3.81-3.92 (m, 1H), 4.05-4.11 (m, 1H), 4.42-4.48 (m, 1H), 4.68-4.70 (m, 1H).

Darstellung von 20G aus 20E:

1.4 g (3.4 mmol) 20E wurden in 30 ml Dioxan gelost. 2 ml 6 N Natronlauge wurden zugetropft. Nach 2 h wurde die Reaktionslösung eingeengt, mit 200 ml Essigester versetzt und auf 200 ml gesättigter Ammoniumchlorid-Lösung gegeben. Diese Mischung wurde mit 1 N Kaliumhydrogensulfat-Lösung auf pH 5 angesäuert und die organische Phase wurde mit gesättigter Natriumchlorid-Lösung gewaschen und mit Natriumsulfat getrocknet. Nach dem Einengen löste man den öligen Rückstand in 15ml wasserfreiem THF und gab 3.0 g (9.5 mmol) Tetrabutylammoniumfluorid (Trihydrat) sowie 0.5 ml Triethylamin zu. Anschließend erwärmte man die Lösung 12 h auf 60°C. Danach engte man die Lösung ein und reinigte den Rückstand an Kieselgel (Laufmittel (Essigester/n-Heptan/Eisessig 30:10:1). Man isolierte 600 mg (54%) 20G als farbloses Öl.

Darstellung von 20H aus 20F:

Analog der Darstellung von 20G aus 20E erhielt man 20H aus 20F.

Darstellung von 20 aus 20H bzw. von 20α aus 20G:

Die Synthese von 20 bzw 20α erfolgte analog der Synthesevorschriften d - f (wie unter 8 beschrieben).
20 : F.p.: 275°C (Zersetzung)
20α: F.p.: 165-175°C (Zersetzung)

Darstellung von Verbindung 21:

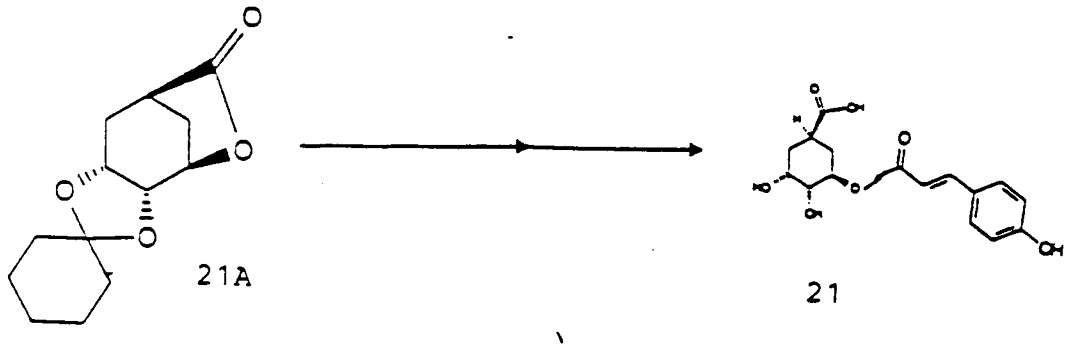

21A

21

Das literaturbekannte Lacton 21A (S.Hanessian, Tetrahedron 45, 6623 (1989)) wurde analog der Synthesevorschriften d - f (wie unter 8 beschrieben) in 21 überführt.F.p.: 227-229°C

Darstellung von 22:

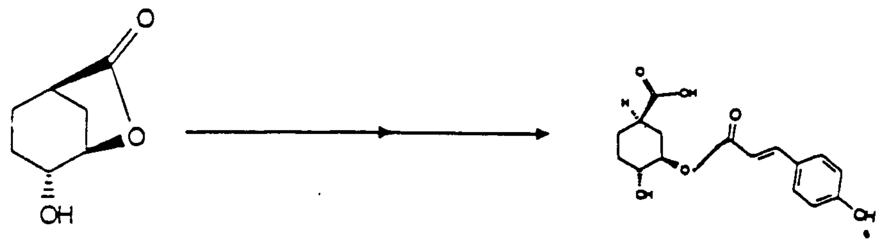

22

22A

Die bekannte Verbindung 22A (S.Mills et al., Tetrahedron Lett. 29, 281 (1988)) wurde analog der Synthesevorschriften d - f (wie unter 8 beschrieben) in 22 überführt. F.p.: 204-206°C

Darstellung von 23:

Formelschema 4

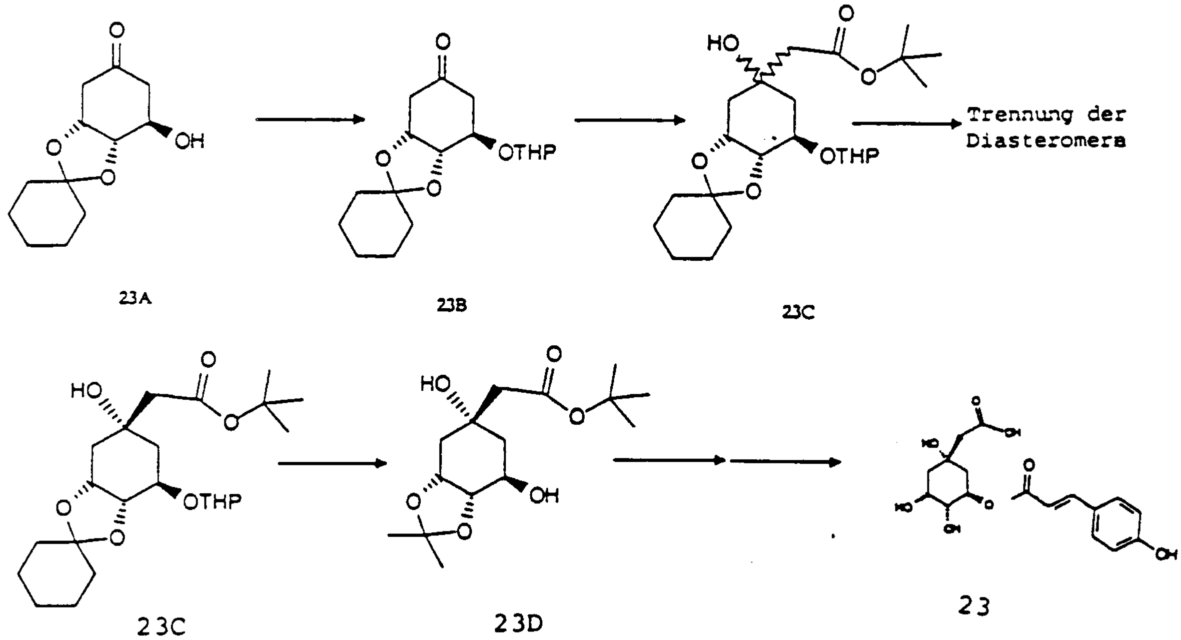


23A 23B 23C

23C 23D 23

THP = Tetrahydropyranoyl

Darstellung von 23B aus 23A:

20.0 g (88.4 mmol)der literaturbekannten Verbindung 23A (J.-C. Barriere et al., Helv.Chim.Acta 66, 296 (1983)) wurden in 200 ml wasserfreiem Dichlormethan gelöst und bei 25°C mit 14.9 g (176.8 mmol) Dihydropyran und 200 mg Pyridinium-p-toluolsulfonat versetzt. Diese Lösung wurde 12 h bei Raumtemperatur gerührt. Anschließend fügte man 500 ml Essigester zu und wusch die organische Phase mit Natriumhydrogencarbonat und gesättigter Natriumchlorid-Lösung. Die organische Phase wurde mit Magnesiumsulfat getrocknet und im Vakuum eingeengt. Man erhielt 26.0 g (95%) 20B als farblosen Feststoff. F.p.: 55-58°C.

Darstellung von 23C aus 23B:

3.66 g (36 mmol) Diisopropylamin wurden in 100 ml wasserfreiem Tetrahydrofuran gelöst. Unter Argon wurden bei -20°C 25 ml 1.5 M n-Butyllithium-Lösung in Hexan zugetropft. Man ließ die Reaktionslösung auf 0°C aufwärmen und kühlte anschließend erneut auf -60°C ab. Bei dieser Tempertur tropfte man 4.1 g (35.3 mmol) Essigsäure-t-butylester gelöst in 20 ml wasserfreiem Tetrahydrofuran langsam zu. Man rührte die Lösung 30 Minuten bei -60°C und tropfte danach 10.0 g (32.2 mmol) 23B gelöst in 30 ml wasserfreiem Tetrahydrofuran bei -60°C zu. Nach einer Stunde Rühren bei gleicher Tempertur hydrolysierte man die Reaktionsmischung mit gesättigter Natriumhydrogencarbonat-Lösung. Man extrahierte mit Essigester und wusch die vereinigten organischen Phasen mit gesättigter Natriumchlorid-Lösung.und trocknete mit Magnesiumsulfat. Nach dem Einengen erhielt man 11.9 g (87%) 23C als hellbraunes Öl.

Darstellung von 23D aus 23C

11.9 g (27.9 mmol) 23C wurden in 200 ml Methanol gelöst. Es wurden 1.8 g Pyridinium-p-toluolsulfonat zugegeben. Man erwärmte 1 h auf Rückflußtemperatur und engte die Reaktionslösung anschließend ein. Den Rückstand löste man in 200 ml wfr. Dichlormethan und gab 8.6 g (93.5mmol) Dimethoxypropan zu. Nach 72 h bei Raumtemperatur wurde die Lösung im Vakuum eingeengt und der Rückstand wurde durch Chromatographie an Kieselgel (Laufmittel Essigester/n-Heptan 1:1) gereinigt. Man erhielt 6.6 g (82%) 23D.

$^{1}$H-NMR (270 MHz, $CDCl_3$) d=1.35 ppm (s, 3H), 1.47 (s, 9H), 1.53 (s, 3H), 1.9-2.12 (m, 1H), 2.22-2.32 (m, 1H),

2.43 (s, 1H), 3.87-3.94 (m, 1H), 4.12-4.25 (m, 1H), 4.35-4.45 (m, 1H).

Darstellung von 23 aus 23D:

Analog der Vorschriften d-f(wie unter 8 beschrieben) erhielt man 23 als farblosen Feststoff. F.p.: 85-92°C

Darstellung von 24:

## Formelschema 5

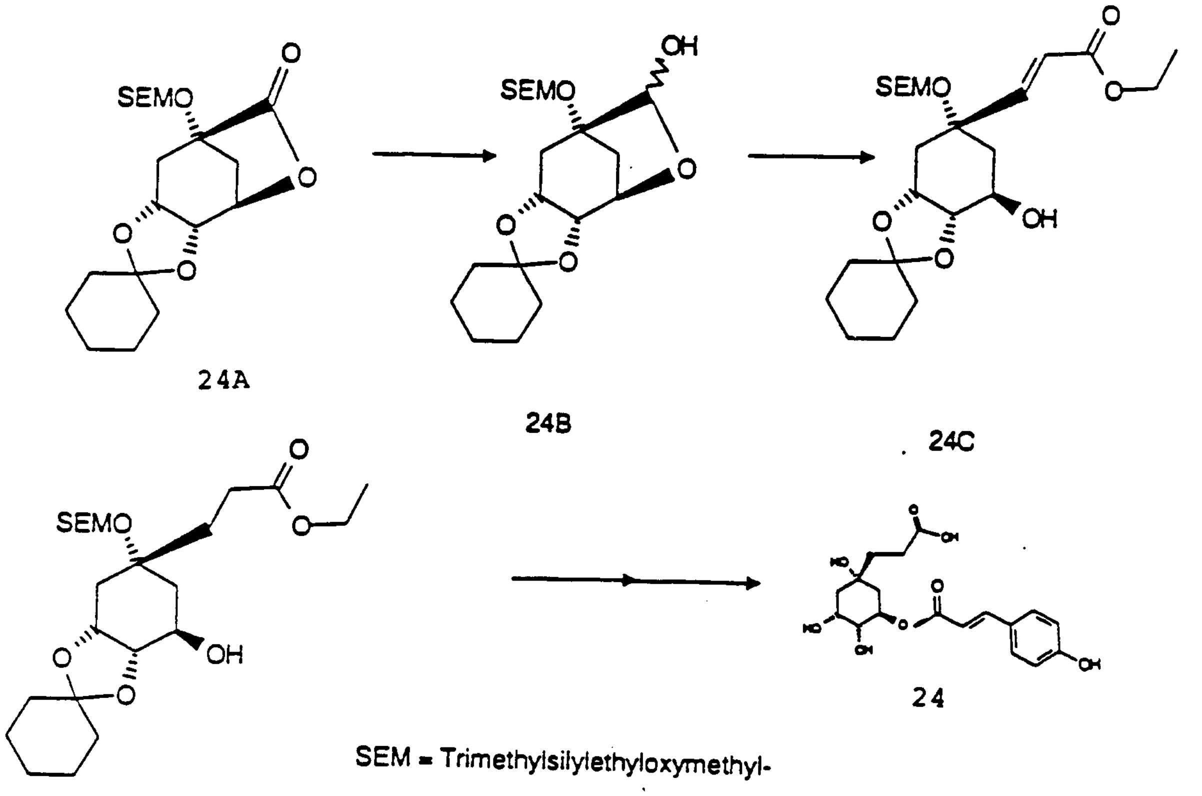

24A

24B

24C

24D

24

SEM = Trimethylsilylethyloxymethyl-

Darstellung von 24B aus 24A:

15.0 g (39 mmol) 24A (J.R.Falck, J.Org. Chem., 54, 5851 (1989)) wurden in 200 ml wasserfreiem Toluol gelöst. Es wurden bei -70°C 38 ml (43 mmol) 1.2 m Diisobutylaluminiumhydrid-Lösung in Hexan zugetropft. Man ließ innerhalb von 2 h auf 0°C aufwärmen und hydrolysierte den Reaktionsansatz mit 10 ml gesättigter Natriumhydrogencarbonat-Lösung. Anschließend gab man nacheinander 10 ml 1 N Natronlauge und 10 ml Wasser zu. Die Reaktionsmischung wurde unter kräftigem Rühren mit 50 g Magnesiumsulfat und 50 g Natriumsulfat versetzt. Man rührte 30 min bei Raumtemperatur, saugte den festen Niederschlag ab und engte das Filtrat ein. Man erhielt 12.9 g (85%) 24B als farbloses Öl, das bei 0°C kristallisierte. F.p.:20-25°C

Darstellung von 24C aus 24B:

Zu einer Suspension von 0.9 g (29.9 mmol) 80%igem Natriumhydrid in 200 ml wasserfreiem Tetrahydrofuran wurden unter Argon-Atmosphäre bei 0°C 7.5 g (33.5 mmol) Phosphonoessigesäuretriethylester zugetropft. Man ließ die Reaktionsmischung langsam auf Raumtemperatur aufwärmen und kühlte die danach klare bräunliche Lösung auf -30°C ab. Man tropfte 7.7 g (19.9 mmol) 24B gelöst in 20 ml wasserfreiem Tetrahydrofuran zu. Diese Lösung wurde 24

h bei -20 bis 30°C gerührt und anschließend mit 100 ml gesättigter Ammoniumchlorid-Lösung versetzt. Man extrahierte mit Essigester, wusch mit gesättigter Natriumchlorid-Lösung und trocknete die vereinigten organischen Phasen mit Magnesiumsulfat. Nach dem Einengen im Vakuum wurde der Rückstand an Kieselgel gereinigt (Laufmittel Essigester/n-Heptan 1:1), und man erhielt 7.5 g (82%) 24C als farbloses Öl.

$^1$H-NMR (200 MHz, $CDCl_3$) : δ=0.01 ppm (s, 9H), 0.85-1.0 (m, 2H), 1.1-1.85 (m, 15H), 2.1-2.25 (m, 2H), 2.35-2.5 (m, 1H), 3.42-3.9 (m, 3H), 4.1-4.4 (m, 4H), 4.65-4.8 (m, 2H), 5.92 (d, J=15 Hz, 1H). MS (FAB): 463.3 ($M+Li^+$).

Darstellung von 24D aus 24C:

1.0 g (2.2 mmol) 24C wurden in 50 ml Essigester gelöst. Es wurden 100 mg $Rh/Al_2O_3$ (5% Rh) zugegeben. Man schüttelte 3 h unter Wasserstoffatmosphäre bei 25°C und Normaldruck. Man filtrierte den Kataylsator ab und engte das Filtrat im Vakuum ein. Man erhielt 0.95 g (94%) 24D als farblosen Feststoff.

Darstellung von 24 aus 24D: Analog der Synthesevorschriften d - f (wie unter 8 beschrieben) erhielt man aus 24D 24. F.p.: 172°C ($H_2O$).

Darstellung von 25:

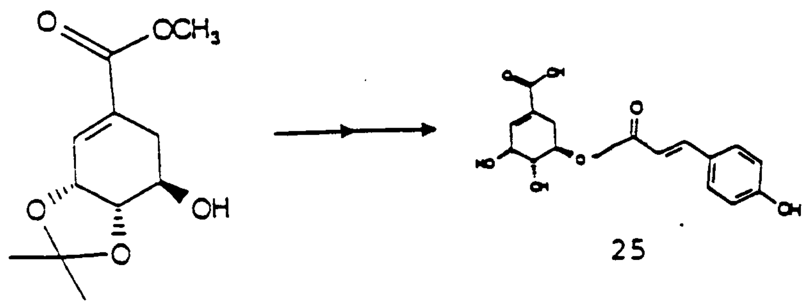

25

25A

Analog der Synthesevorschriften d - f (wie unter 8 beschrieben) erhielt man aus der literaturbekannten Vorstufe 25A (J.L.Pawlak et al., J.Org.Chem. 52, 1765 (1987)) die Verbindung 25. F.p.: 75-80°C (Schäumen)

Darstellung von 26 aus 26A:

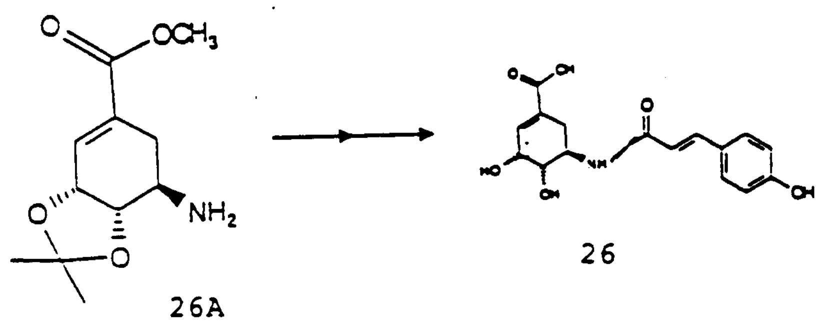

26

26A

Analog der Synthesevorschriften d - f (wie unter 8 beschrieben) erhielt man aus der literaturbekannten Vorstufe 26A die Verbindung 26 als farblosen amorphen Feststoff.

$^1$H-NMR (270 MHz, $d_6$-DMSO): d= 1.95-2.14 ppm (m, 1H), 2.55-2.70 (m, 1H), 3.62-3.76 (m, 1H), 4.08-4.26 (m, 2H), 4.55-4.75 (m, 1H), 4.9-5.1 (m, 1H), 6.48 (d, J=10.0 Hz, 1H), 6.63-6.72 (m, 1H), 6.75-6.88 (m, 2H), 7.29-7.46 (m, 3H), 7.89 (d, J=5 Hz, 1H), 9.70- 10.0 (1H), 12.2-12.6 (1H). MS (CI): 225.2 ($M+H^+$).

Darstellung von 27 und 28:

Formelschema 6

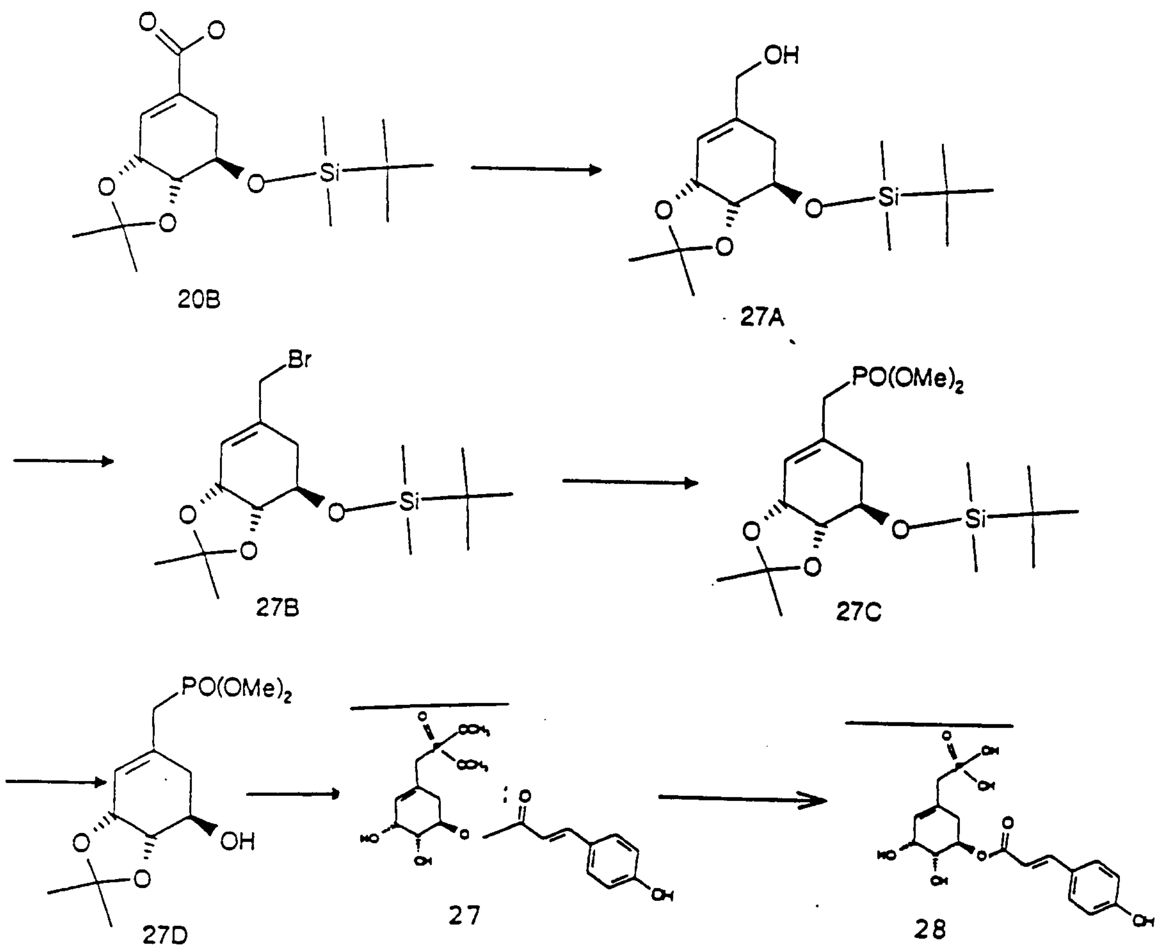

20B 27A 27B 27C 27D 27 28

Darstellung von 27A aus 20B:

6.0 g (17.5 mmol) 20B wurden in 100 ml wasserfreiem Toluol gelöst. Es wurden bei -20°C 29.2 ml 1.2 M Diisobutylaluminiumhydrid-Lösung in Hexan zugetropft. Man ließ innerhalb von 1 h auf 25°C aufwärmen, kühlte erneut auf 0°C ab und tropfte vorsichtig 20 ml einer 9:1-Mischung Methanol/Wasser zu. Anschließend tropfte man weiter 30 ml einer gesättigten Ammoniumchlorid-Lösung zu und rührte die Reaktionsmischung 30 min. bei 25°C. Danach wurde mit Essigester extrahiert und die vereinigten organischen Phasen wurden mit gesättigter Natriumchlorid-Lösung gewaschen, mit Magnesiumsulfat getrocknet und im Vakuum eingeengt. Den Rückstand reinigte man durch Chromatographie an Kieselgel (Essigester/n-Heptan 1:3). Man erhielt 3.5 g (63%) 27B als farbloses Öl.

$^1$H-NMR (270 MHz, $CDCl_3$): d= 0.08 ppm (s, 3H), 0.11 (s, 3H), 0.89 (s, 9H), 1.39 (s, 3H), 1.46 (s, 3H), 1.97-2.09 (m, 1H), 2.19-2.30 (m, 1H), 3.88-3.92 (m 1H), 3.98-4.09 (m, 4H), 4.62-4.68 (m, 1H), 5.76-5.82 (m, 1H).

Darstellung von 27B aus 27A:

Zu einer Lösung von 2.9 g (16.2 mmol) N-Bromsuccinimid in 100 ml wasserfreiem Dichlormethan wurden bei 0°C 1.43 ml (19.6 mmol) Dimethylsulfid zugetropft. Nach 5 min. kühlte man auf -20°C ab und tropfte 3.4 g (10.8 mmol) 27A gelöst in 20 ml wasserfreiem Dichlormethan zu. Anschließend wurde die hellgelbe Suspension langsam auf 25°C aufgewärmt und 3 h gerührt. Anschließend versetzte man mit 100 ml gesättigter Ammoniumchlorid-Lösung und extrahierte mit 500 ml Essigester. Die vereinigten organischen Phasen wurden mit gesättigter Natriumchlorid-Lösung gewaschen und mit Magnesiumsulfat getrocknet. Nach dem Einengen wurde der Rückstand durch Chromatographie an Kieselgel gereinigt (Laufmittel Essigester/Heptan 1:3) und man erhielt 3,7 g (98%) 27B als farbloses Öl.

1H-NMR (270 MHz, CDCl3) δ= 0.09 ppm (s, 3H), 0.10 (s, 3H), 0.89 (s, 9H), 1.38 (s, 3H), 1.41 (s, 3H), 2.09-2.21 (m, 1H), 2.35-2.45 (m, 1H), 3.92 (s, 2H), 3.97-4.05 (m, 2H), 4.38-4.65 (m, 1H), 5.83-5.89 (m, 1H).

MS (Cl): 377.1 ($M+H^+$).

Darstellung von 27C aus 27B:

3.0 g (7.6 mmol) 27B wurden in 42 ml Trimethylphosphit 6 h auf 90°C erwärmt. Anschließend destillierte man das überschüssige Phosphit im Vakuum ab und reinigte den Rückstand durch Chromatographie an Kieselgel (Laufmittel Essigester/Methanol 5:1). Es wurden 3.0 g (93%) 27C als farbloses Öl erhalten.

Darstellung von 27D aus 27C:

3.0 g (7.4 mmol) 27C wurden in 50 ml Methanol gelöst. Es wurde 1 ml 1N Salzsäure zugegeben. Nach 24 h neutralisierte man die Reaktionslösung mit 1 N Natronlauge und engte im Vakuum bis zur Trockne ein. Den Rückstand nahm man in 50 ml wasssertreiem Dichlormethan auf, gab 5 ml Dimethoxypropan sowie 0.5 g Pyridinium-p-toluolsulfonat zu und erhitzte 4 h auf 40°C. Danach gab man die Lösung auf gesättigte Natriumhydrogencarbonat-Lösung und extrahierte mit 500 ml Essigester. Die vereinigten organischen Phasen wurden mit gesättigter Natriumchlorid-Lösung gewaschen, mit Magnesiumsulfat getrocknet und im Vakuum eingeengt. Den Rückstand reinigte man durch Chromatographie an Kieselgel (Laufmittel Essigester/Methanol 10:1) und erhielt 1.5 g (70%) 27D als farbloses Öl.

Darstellung von 27 aus 27D

Analog der Synthesevorschriften e-f (wie unter 8 beschrieben) wurde aus 27D die Verbindung 27 erhalten.

$^1$H-NMR (200 MHz, $d_6$-DMSO): δ= 2.05-2.22 ppm (m, 1H), 2.55-2.8 (m, 1H), 3.4-3.55 (m, 1H), 3.6 (s, 3H), 3.65 (s, 3H), 4.05-4.15 (m, 1H), 4.3-4.4 (m, 1H), 4.6-4.8 (m, 3H), 5.0-5.15 (m, 1H), 5.55-5.68 (m, 1H), 6.3-6.45 (m, 1H), 6.37-6.45 (m, 2H), 7.5-7.7 (m, 3H), 10.0 (s, 1H).

MS (Cl): 399 ($M^+$), 381 ($M^+$-$H_2O$).

Darstellung von 28 aus 27:

135 mg (0.34 mmol) 27 wurden in 10 ml wasserfreiem Acetonitril gelöst. Bei 0°C wurden 155 mg (1 mmol) Trimethylsilylbromid zugetropft.Man rührte 30 Minuten und gab anschließend 5 ml Wasser zu. Man versetzte mit 1 N Natronlauge bis etwa pH 5 erreicht wurde und engte im Vakuum ein. Der Rückstand wurde durch Chromatographie an RP-8-Kieselgel gereinigt (Laufmittel Wasser/Methanol 4:1) und man erhielt 23 mg (18%) 28 als farblosen Feststoff. F.p.: 180-185°C.

Die pharmazeutischen Präparate werden nach allgemein üblichen Verfahren hergestellt.

Beispiel 1 Tabletten

Tabletten, die für die orale Verabreichung geeignet sind und die nachfolgend genannten Bestandteile enthalten, wurden auf an sich bekannte Weise hergestellt, indem man Wirk- und Hilfsstoffe granulierte und anschließend zu Tabletten verpreßte.

| Bestandteile (pro Tablette) | Gewicht (mg) |
|---|---|
| Verbindung der Formel I (z.B. Verbindung 17) | 50 mg |
| Milchzucker | 100 mg |
| Maisstärke | 30 mg |
| Talkum | 3 mg |
| kolloidales Siliziumdioxid | 3 mg |
| Magnesiumstearat | 2 mg |

Beispiel 2: Kapseln

Kapseln, die für die orale Verabreichung geeignet sind, enthielten die nachfolgend genannten Bestandteile und wurden auf an sich bekannte Weise hergestellt, indem man Wirk- und Hilfsstoffe vermischte und in Gelatinekapseln abfüllte.

| Bestandteile (pro Kapsel) | Gewicht (mg) |
|---|---|
| Verbindung der Formel I (z.B. Verbindung 21) | 50 mg |
| Milchzucker | 100 mg |
| Maisstärke | 30 mg |
| Talkum | 3 mg |
| kolloidales Siliziumdioxid | 3 mg |
| Magnesiumstearat | 2 mg |

**Patentansprüche**

1. Anwendung von Cyclohexan-Derivaten der Formel I

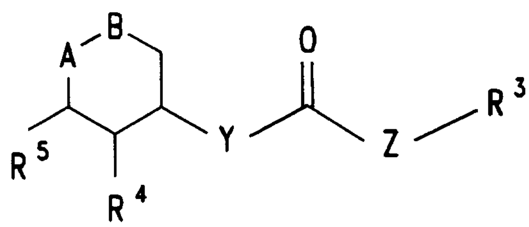

I

worin

A-B die Gruppe 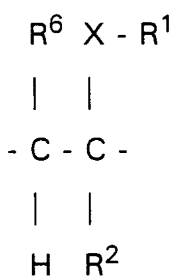

oder

die Gruppe 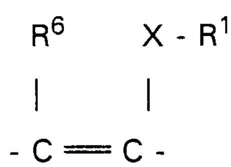

bedeutet,

$R^1$: CN, COOH, COO-($C_1$-$C_4$-Alkyl), $C_1$-$C_4$-Alkanoyl, $SO_3$-($C_1$-$C_4$-Alkyl), $SO_3H$, $PO(OH)_2$, PO(OH)(O-$C_1$-$C_4$-Alkyl) oder PO(O-$C_1$-$C_4$-Alkyl$)_2$ ist,

$R^2$: H, OH oder F ist,

$R^3$: H, Phenyl, Naphthyl, Pyridyl, Thienyl, Furyl ist, wobei der Aromat bzw. Heteroaromat ein- oder mehrfach mit F, Cl, Br, I, OH, $NO_2$, $C_1$-$C_4$-Alkanoyl, $C_1$-$C_4$-Alkoxy, $C_1$-$C_4$-Alkyl, Phenyl, Phenoxy, Thienyl, Furyl, Pyridyl, Imidazolyl oder Benzyloxy substituiert sein kann, wobei die Substituenten gleich oder verschieden sind,

$R^4$, $R^5$, $R^6$: H, OH, F, Cl, Br, $C_1$-$C_4$-Alkanoyl, $C_1$-$C_4$-Alkyl, Phenyl, Phenoxy, Thienyl, Furyl, Pyridyl, Imidazolyl oder Benzyloxy bedeuten, wobei $R^4$, $R^5$, $R^6$ gleich oder verschieden sind,

X: $-(CH_2)_n-$, -CH = CH- oder $-CH_2OCH_2-$ ist,

Y: $-(CH_2)_n-$, O, S oder NH ist,

Z: $-(CH_2)_n-$ oder -CH = CH- ist und

n: null, 1, 2, 3 oder 4 ist

zur Herstellung von Arzneimitteln zur Behandlung des Typ II Diabetes und anderer Erkrankungen, die durch einen erhöhten Glucose-Ausstoß aus der Leber oder eine erhöhte Aktivität des Glucose-6-Phosphatase-Systems gekennzeichnet sind.

**2.** Anwendung von Cyclohexan-Derivaten der Formel I nach Anspruch 1, in denen die Reste in Formel I die folgende Bedeutung haben:

$R^1$: COOH, COO-($C_1$-$C_4$-Alkyl), $PO(OH)_2$, PO(OH)(O-$C_1$-$C_4$-Alkyl) oder PO(O-$C_1$-$C_4$-Alkyl)$_2$,

$R^2$: H oder OH

$R^3$: H, Phenyl, Naphthyl, Pyridyl, Thienyl, Furyl, wobei der Aromat bzw. Heteroaromat ein-, zwei- oder dreifach mit F, Cl, Br, I, $NO_2$, OH, $C_1$-$C_4$-Alkanoyl, $C_1$-$C_4$-Alkoxy, $C_1$-$C_4$-Alkyl, Phenyl, Phenoxy, Thienyl, Furyl, Pyridyl, Imidazolyl oder Benzyloxy substituiert sein kann, wobei die Substituenten gleich oder verschieden sind,

$R^4$, $R^5$ u. $R^6$ : H, OH, F, Cl, Br, $C_1$-$C_4$-Alkanoyl, $C_1$-$C_4$-Alkyl, Phenyl, Phenoxy, Thienyl, Furyl, Pyridyl, Imidazolyl oder Benzyloxy, wobei $R^4$, $R^5$, $R^6$ gleich oder verschieden sind,

X: $-(CH_2)_n-$, -CH = CH- oder $-CH_2OCH_2-$,

Y: $-(CH_2)_n-$, O, S oder NH,

Z: $-(CH_2)_n-$ oder -CH = CH- und

n: null, 1, 2, 3 oder 4

zur Herstellung von Arzneimitteln zur Behandlung des Typ II Diabetes und anderer Erkrankungen, die durch einen erhöhten Glucose-Ausstoß aus der Leber oder eine erhöhte Aktivität des Glucose-6-Phosphatase-Systems gekennzeichnet sind.

**3.** Anwendung von Cyclohexan-Derivaten der Formel I nach Anspruch 1, in denen die Reste in Formel I die folgende Bedeutung haben:

$R^1$: COOH oder COO-($C_1$-$C_4$-Alkyl)

$R^2$: H oder OH,

$R^3$: H, Phenyl, Naphthyl, Pyridyl, Thienyl, Furyl, wobei der Aromat bzw. Heteroaromat ein-, zwei- oder dreifach mit F, Cl, OH, $NO_2$, $C_1$-$C_4$-Alkanoyl, $C_1$-$C_4$-Alkoxy, $C_1$-$C_4$-Alkyl, Phenyl, Phenyloxy oder Benzyloxy gleich oder verschieden substituiert sein kann,

$R^4$, $R^5$ und $R^6$ : H oder OH, wobei $R^4$, $R^5$, $R^6$ gleich oder verschieden sind,

X: $-(CH_2)_n-$ und n = null, 1 oder 2

Y: O oder NH,

Z: $-(CH_2)_n-$ mit n = 0 oder 2 oder -CH = CH-,

zur Herstellung von Arzneimitteln zur Behandlung des Typ II Diabetes und anderer Erkrankungen, die durch einen erhöhten Glucose-Ausstoß aus der Leber oder eine erhöhte Aktivität des Glucose-6-Phosphatase-Systems gekennzeichnet sind.

## Claims

**1.** The use of cyclohexane derivatives of the formula I

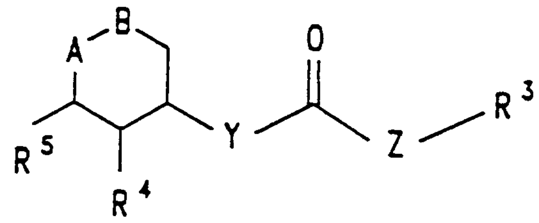

in which

A-B is the group 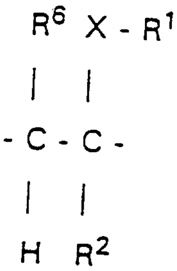

or

the group 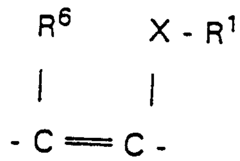 ,

| | |
|---|---|
| $R^1$ is: | CN, COOH, COO-($C_1$-$C_4$-alkyl), $C_1$-$C_4$-alkanoyl, $SO_3$-($C_1$-$C_4$-alkyl), $SO_3H$, $PO(OH)_2$, PO(OH)(O-$C_1$-$C_4$-alkyl) or PO(O-$C_1$-$C_4$-alkyl)$_2$, |
| $R^2$ is: | H, OH or F, |
| $R^3$ is: | H, phenyl, naphthyl, pyridyl, thienyl or furyl, where the aromatic or heteroaromatic system can be monosubstituted or polysubstituted by F, Cl, Br, I, OH, $NO_2$, $C_1$-$C_4$-alkanoyl, $C_1$-$C_4$-alkoxy, $C_1$-$C_4$-alkyl, phenyl, phenoxy, thienyl, furyl, pyridyl, imidazolyl or benzyloxy, where the substituents are identical or different, |
| $R^4$, $R^5$ and $R^6$ are: | H, OH, F, Cl, Br, $C_1$-$C_4$-alkanoyl, $C_1$-$C_4$-alkyl, phenyl, phenoxy, thienyl, furyl, pyridyl, imidazolyl or benzyloxy, where $R^4$, $R^5$ and $R^6$ are identical or different, |
| X is: | $-(CH_2)_n-$, -CH=CH- or $-CH_2OCH_2-$, |
| Y is: | $-(CH_2)_n-$, O, S or NH, |
| Z is: | $-(CH_2)_n-$ or -CH=CH- and |
| n is: | zero, 1, 2, 3 or 4 |

for the production of pharmaceuticals for the treatment of type II diabetes and other disorders which are characterized by an increased glucose discharge from the liver or an increased activity of the glucose-6-phosphatase system.

2. The use of cyclohexane derivatives of the formula I as claimed in claim 1, in which the radicals in formula I have the following meaning:

| | |
|---|---|
| $R^1$ is: | COOH, COO-($C_1$-$C_4$-alkyl), $PO(OH)_2$, PO(OH)(O-$C_1$-$C_4$-alkyl) or PO(O-$C_1$-$C_4$-alkyl)$_2$, |
| $R^2$ is: | H or OH, |
| $R^3$ is: | H, phenyl, naphthyl, pyridyl, thienyl or furyl, where the aromatic or heteroaromatic system can be monosubstituted, disubstituted or trisubstituted by F, Cl, Br, I, $NO_2$, OH, $C_1$-$C_4$- |

alkanoyl, $C_1$-$C_4$-alkoxy, $C_1$-$C_4$-alkyl, phenyl, phenoxy, thienyl, furyl, pyridyl, imidazolyl or benzyloxy, where the substituents are identical or different,

$R^4$, $R^5$ and $R^6$ are: H, OH, F, Cl, Br, $C_1$-$C_4$-alkanoyl, $C_1$-$C_4$-alkyl, phenyl, phenoxy, thienyl, furyl, pyridyl, imidazolyl or benzyloxy, where $R^4$, $R^5$ and $R^6$ are identical or different,

X is: $-(CH_2)_n-$, -CH=CH- or $-CH_2OCH_2-$,

Y is: $-(CH_2)_n-$, O, S or NH,

Z is: $-(CH_2)_n-$ or -CH=CH- and

n is: zero, 1, 2, 3 or 4

for the production of pharmaceuticals for the treatment of type II diabetes and other disorders which are characterized by an increased glucose discharge from the liver or an increased activity of the glucose-6-phosphatase system.

**3.** The use of cuclohexane derivatives of the formula I as claimed in claim 1, in which the radicals in formula I have the following meaning:

$R^1$ is: COOH or COO-($C_1$-$C_4$-alkyl),

$R^2$ is: H or OH,

$R^3$ is: H, phenyl, naphthyl, pyridyl, thienyl or furyl, where the aromatic or heteroaromatic system can be monosubstituted, disubstituted or trisubstituted by identical or different F, Cl, OH, $NO_2$, $C_1$-$C_4$-alkanoyl, $C_1$-$C_4$-alkoxy, $C_1$-$C_4$-alkyl, phenyl, phenyloxy or benzyloxy,

$R^4$, $R^5$ and $R^6$ are: H or OH, where $R^4$, $R^5$ and $R^6$ are identical or different,

X is: $-(CH_2)_n-$ and n = zero, 1 or 2,

Y is: O or NH,

Z is: $-(CH_2)_n-$, where n = 0 or 2, or -CH=CH-

for the production of pharmaceuticals for the treatment of type II diabetes and other disorders which are characterized by an increased glucose discharge from the liver or an increased activity of the glucose-6-phosphatase system.

**Revendications**

**1.** Utilisation de dérivés de cyclohexane de formule I

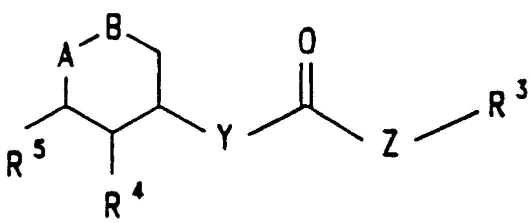

I

dans laquelle

A-B représente le groupe 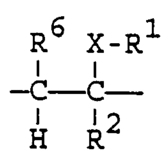

ou le groupe 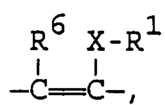,

$R^1$ représente un groupe CN, COOH, COO-alkyle($C_1$-$C_4$), alcanoyle en $C_1$-$C_4$, $SO_3$-alkyle($C_1$-$C_4$),

$SO_3H$, $PO(OH)_2$, PO(OH)[O-alkyle($C_1$-$C_4$)] ou PO[O-alkyle($C_1$-$C_4$)]$_2$,

$R^2$ est H, OH ou F,

$R^3$ est H ou le groupe phényle, naphtyle, pyridyle, thiényle, furyle, le fragment aromatique ou hétéroaromatique pouvant être une ou plusieurs fois substitué par F, Cl, Br, I, OH, $NO_2$, un groupe alcanoyle en $C_1$-$C_4$, alcoxy en $C_1$-$C_4$, alkyle en $C_1$-$C_4$, phényle, phénoxy, thiényle, furyle, pyridyle, imidazolyle ou benzyloxy, les substituants pouvant être identiques ou différents,

$R^4$, $R^5$, $R^6$ représentent H, OH, F, Cl, Br ou un groupe alcanoyle en $C_1$-$C_4$, alkyle en $C_1$-$C_4$, phényle, phénoxy, thiényle, furyle, pyridyle, imidazolyle ou benzyloxy, $R^4$, $R^5$, $R^6$ pouvant être identiques ou différents,

X est $-(CH_2)_n-$, -CH=CH- ou $-CH_2OCH_2-$,

Y est $-(CH_2)_n-$, O, S ou NH,

Z est $-(CH_2)_n-$ ou -CH=CH- et

n est égal à zéro, 1, 2, 3 ou 4,

pour la fabrication de médicaments destinés au traitement du diabète de type II et d'autres maladies, qui sont caractérisées par une production accrue de glucose par le foie ou une activité accrue du système glucose-6-phosphatase.

**2.** Utilisation de dérivés de cyclohexane de formule I selon la revendication 1, dans lesquels les radicaux dans la formule I ont les significations suivantes:

$R^1$ représente un groupe COOH, COO-alkyle($C_1$-$C_4$), $PO(OH)_2$, PO(OH)[O-alkyle($C_1$-$C_4$)] ou PO[O-alkyle($C_1$-$C_4$)]$_2$,

$R^2$ est H ou OH,

$R^3$ représente H ou le groupe phényle, naphtyle, pyridyle, thiényle, furyle, le fragment aromatique ou hétéroaromatique pouvant être substitué une, deux ou trois fois par F, Cl, Br, I, $NO_2$, OH, un groupe alcanoyle en $C_1$-$C_4$, alcoxy en $C_1$-$C_4$, alkyle en $C_1$-$C_4$, phényle, phénoxy, thiényle, furyle, pyridyle, imidazolyle ou benzyloxy, les substituants pouvant être identiques ou différents,

$R^4$, $R^5$ et $R^6$ représentent H, OH, F, Cl, Br, un groupe alcanoyle en $C_1$-$C_4$, alkyle en $C_1$-$C_4$, phényle, phénoxy, thiényle, furyle, pyridyle, imidazolyle ou benzyloxy, $R^4$, $R^5$, $R^6$ pouvant être identiques ou différents,

X est $-(CH_2)_n-$, -CH=CH- ou $-CH_2OCH_2-$,

Y est $-(CH_2)_n-$, O, S ou NH,

Z est $-(CH_2)_n-$ ou -CH=CH- et

n est égal à zéro, 1, 2, 3 ou 4,

pour la fabrication de médicaments destinés au traitement du diabète de type II et d'autres maladies qui sont caractérisées par une production accrue de glucose par le foie ou une activité accrue du système glucose-6-phosphatase.

**3.** Utilisation de dérivés de cyclohexane de formule I selon la revendication 1, dans lesquels les radicaux dans la formule I ont les significations suivantes:

$R^1$ représente un groupe COOH ou COO-alkyle($C_1$-$C_4$),

$R^2$ est H ou OH,

$R^3$ représente H ou le groupe phényle, naphtyle, pyridyle, thiényle, furyle, le fragment aromatique ou hétéroaromatique pouvant être substitué une, deux ou trois fois par F, Cl, OH, $NO_2$, un groupe alcanoyle en $C_1$-$C_4$, alcoxy en $C_1$-$C_4$, alkyle en $C_1$-$C_4$, phényle, phényloxy ou benzyloxy, les substituants pouvant être identiques ou différents,

$R^4$, $R^5$ et $R^6$ représentent H ou OH, $R^4$, $R^5$, $R^6$ pouvant être identiques ou différents,

X est $-(CH_2)_n-$ et n est égal à zéro, 1 ou 2,

Y est O ou NH,

Z est $-(CH_2)_n-$, avec n = zéro ou 2 ou -CH=CH-,

pour la fabrication de médicaments destinés au traitement du diabète de type II et d'autres maladies qui sont caractérisées par une production accrue de glucose par le foie ou une activité accrue du système glucose-6-phosphatase.